# FASCICULE DE BREVET EUROPEEN

(11) **EP 2 048 238 B1**
(45) Date de publication et mention de la délivrance du brevet: **25.01.2012**
(21) Numéro de dépôt: 08305671.3
(22) Date de dépôt: 10.10.2008
(51) Int. Cl.: A23L 1/30, A23L 1/03, A61K 8/97, A61Q 19/00, C12P 19/00, C12P 19/14

(54) **Procédé de préparation d'un extrait végétal**
Verfahren zur Herstellung eines Pflanzenextrakts
Method for preparing a plant extract

(30) Priorité: 12.10.2007 FR 0758257
(43) Date de publication de la demande: 15.04.2009
(73) Titulaire: Thiomed, 03800 Saint Bonnet de Rochefort (FR)
(72) Inventeur: Dubourdeaux, Michel, 03140 TAXAT (FR)
(74) Mandataire: Thivillier, Patrick

(56) Documents cités:
- EP-A- 0 381 055
- EP-A- 0 425 391
- DATABASE WPI Week 198530 Thomson Scientific, London, GB; AN 1985-181169 XP002470996 -& JP 60 109526 A (HASEGAWA CO LTD) 15 juin 1985 (1985-06-15)
- SAKAMOTO K ET AL: "Decreased hardness of dietary fiber-rich foods by the enzyme-infusion method" BIOSCIENCE, BIOTECHNOLOGY, AND BIOCHEMISTRY, vol. 70, no. 7, 23 juillet 2006 (2006-07-23), pages 1564-1570, XP002470991

## Description

L'invention a pour objet un nouveau procédé de préparation d'un extrait végétal.

Les matières premières végétales utilisables en industrie sont obtenues par extraction à partir d'un matériel végétal donné (partie de plante ou plante entière). De nombreux procédés d'extraction sont connus tels que notamment macération en présence d'un solvant, dessiccation, broyage etc... Les extraits obtenus se présentent soit sous forme liquide (teintures, extraits fluides, ou dérivés), soit sous forme solide (par exemple extrait sec séché sur des supports type maltodextrines). On peut considérer aujourd'hui que l'ensemble des matières premières végétales entrant dans la composition des compléments alimentaires, cosmétiques, ou pharmaceutiques sont issus des modes de transformation cités ci-dessus. Ces différentes techniques requièrent notamment la mise en oeuvre de solvants spécifiques et l'application de températures élevées pour garantir une concentration des substances actives.

Les documents FR--2 443 266 et FR--2 443 256 décrivent respectivement un procédé de fabrication d'un extrait de ginseng et de matricaire consistant à faire réagir la matière première avec une solution enzymatique. La matière végétale est mise au contact de la solution enzymatique, à pression atmosphérique, pendant 2 à 4 heures. Le mélange est ensuite additionné de solvant, en l'espèce de propylène glycol, puis mis à macérer pendant 1 semaine.

Le document JP60109526 décrit un procédé de fabrication d'un extrait de plante consistant à faire réagir une poudre de plante congelée, avec une solution enzymatique, à pression atmosphérique.

Le document JP2291243 décrit un procédé de fabrication d'une solution contenant un extrait de pomme de terre sucrée consistant à mettre en contact la pomme de terre avec une solution enzymatique, à pression atmosphérique.

Le document JP 1300848 décrit une boisson à base de thé. Le procédé d'extraction consiste à mettre en contact les feuilles de thé avec une solution enzymatique, sous gaz inerte. Aucune information sur la pression appliquée n'est donnée. Cependant, du fait de la nature du produit fini (boisson), il est probable que le procédé soit conduit à pression atmosphérique.

Le document JP61289853 décrit un procédé de fabrication d'un extrait de ginseng obtenu par digestion enzymatique de plantes fraiches, à pression atmosphérique. Pour favoriser l'extraction, la plante est mise à bouillir pendant 10 minutes avant le traitement enzymatique.

Le document EP 0425391 décrit une méthode d'extraction de plantes par broyages successifs de plantes entières puis de fractions de plantes à température comprise entre - 40 et -100°C. Les fractions sont ensuite tamisées puis pressées mécaniquement pour atteindre une température de l'ordre de 0 à 5°C. Par cycles successifs, les fractions pressées sont ensuite congelées puis remises en suspension à température de 0 à 5°C. Le liquide obtenu est enfin filtré.

Le document Sakamoto et al., Biosci. Biotechnol. Biochem. 70(7), 1564-1570 (2006), décrit un procédé de ramollissement de tissus de plantes consistant à faire macérer une poudre congelée de plante avec des enzymes de digestion en solution à 40°C. La digestion est effectuée sous atmosphère réduite pendant 5 minutes puis le mélange est remis à pression atmosphérique. Dans ce document, la digestion n'est que partielle, l'objectif étant de ramollir les végétaux pour faciliter leur ingestion par les personnes agées.

Les inconvénients découlant de ces conditions de mise en oeuvre sont notamment :
- l'extraction d'une partie seulement des principes actifs en fonction de la nature du solvant choisi, extraction sélective qui est incompatible avec la législation des compléments alimentaires,
- l'absence de conservation de l'intégralité et de l'intégrité des molécules par élévation de la température notamment,
- la présence de composés cellulosiques venant diminuer la concentration en substance active ainsi que leur biodisponibilité.

Le problème que se propose de résoudre l'invention est donc de mettre au point un procédé d'extraction respectant en tout point l'intégrité et l'intégralité des composants végétaux, tant d'un point de vue physicochimique que d'un point de vue organoleptique (odeur, couleur, goût) et ce, sans sélection aucune des principes actifs contenus dans la matière végétale de départ.

Un autre objectif de l'invention est de mettre au point un procédé permettant d'obtenir des extraits secs ou liquides pouvant être facilement incorporables dans des formes galéniques diverses comme des gélules, comprimés, sachets ou des liquides, sirop, boissons, voire des préparations alimentaires.

La première différence remarquable entre la cellule animale et la cellule végétale réside en ce que cette dernière est emprisonnée dans une paroi rigide faite d'un matériau typiquement végétal, la cellulose. Cette paroi, désignée paroi pectocellulosique confère à la plante des propriétés uniquement mécaniques, assurant par ailleurs une action physiologique limitée de lest. Outre la cellulose, cette paroi, qui représente une masse importante au sein du végétal, contient également de la pectine, de l'hémicellulose, de l'eau, des enzymes et des protéines de structure.

Le Demandeur a constaté que de manière tout à fait surprenante, le fait d'effectuer la digestion des tissus végétaux selon un cycle de pression/dépression, sous gaz inerte, permettait, permettait, en combinaison avec d'autres étapes connues en elles mêmes, d'obtenir un extrait remplissant tous les objectifs ci-avant évoqués.

L'invention a donc pour objet un procédé de préparation d'un extrait végétal, comprenant les étapes suivantes :
- refroidissement de la plante ou partie de plante fraiche, en particulier les parties aériennes à une température comprise entre -10 et -100°C,
- broyage de la plante congelée,
- mise en contact de la plante congelée broyée avec partie d'une solution enzymatique apte à digérer la paroi pectocellulosique des cellules végétales,
- ajustement du pH au pH auquel agissent les enzymes, de même que de la température à la température à laquelle agissent les enzymes,
- ajout du reste de solution enzymatique,
- application d'un cycle de dépression et de pression au moyen d'un gaz inerte,
- une fois la digestion terminée, tamisage puis stabilisation de l'extrait liquide obtenu.

Selon une première caractéristique, la matière végétale de départ est placée dans une enceinte au contact d'azote liquide ou tout autre gaz inerte, afin de stabiliser ce matériel et dans le cas de l'azote, de l'amener à une température comprise entre -10° et -80°C. Avantageusement, la plante est refroidie à une température comprise entre -60 et -70°C.

Selon l'invention, la matière végétale est broyée, avantageusement sous azote, après congélation et se présente sous la forme d'une poudre de granulométrie comprise entre 2 cm et 100 µm, avantageusement entre 100 et 500 µm, c'est-à-dire une taille supérieure à celle des cellules. Bien entendu, toute technique adéquate peut être mise en oeuvre pour obtenir une poudre, telle que notamment le broyage, à l'aide par exemple d'un cutter, sous vide ou tout autre moyen de broyage.

La matière végétale broyée congelée est mise en contact dans un premier temps avec une partie seulement de la solution enzymatique désignée encore «pied de cuve enzymatique ». C'est notamment le cas lorsque la matière végétale se présente sous la forme de fruits frais riches en eau. Lorsque la matière végétale se présente sous la forme de parties de plantes autres que les fruits, présentant une teneur faible en eau, la matière végétale est dispersée au sein d'un mélange d'eau et de solution enzymatique. Il s'agit d'une étape de mouillage effectuée sous agitation pendant une durée d'environ 10 mn, en fonction du volume de matière à traiter. Avantageusement, l'eau représente 1 à 2 fois le volume de matière végétale.

Avant l'étape de digestion à proprement parler, il convient d'ajuster le pH du milieu au pH auquel agissent les enzymes assurant la lyse de la paroi pectocellulosique, de même que la température du milieu à laquelle agissent les enzymes de digestion, en pratique par prélèvement d'un échantillon.

Bien entendu, le pH et la température vont dépendre de la nature des enzymes utilisées. Il peut s'agir de pectinases, lignases, celluloses, β glucanases. De manière générale, la température est comprise entre 25 et 40°C et le pH compris entre 3 et 6.

Le chauffage permettant de faire passer la température de la matière végétale d'environ - 20°C (à l'issue du transfert de la matière congelée, en pratique, dans le turbosphère) à plus de 25°C est effectué par convection ou hyperfréquence, ce qui permet d'atteindre la température adéquate en un temps très court. Cette étape de chauffage rapide contribue également au rendement de l'extraction.

Une fois les ajustements de température et de pH effectués, an ajoute au pied de cuve enzymatique le reste de la solution. La digestion peut alors être accélérée par application d'une dépression d'une valeur d'au moins 5600 Pa (42 mm Hg). Cette dépression alterne avec un cycle de mise en pression à une valeur maximale de 2 bars, c'est-à-dire 1 bar au dessus de la pression atmosphérique. Selon, une caractéristique essentielle, cette mise en pression est effectuée au moyen d'un gaz inerte tel que azote, l'argon ou autre gaz rares ou hélium.

La durée de l'étape de digestion dépend de nombreux paramètres tels que notamment la granulométrie, la température et le pH. En pratique la digestion dure entre 30 mn à plusieurs heures.

Pour être capable de vérifier dans quelle mesure la digestion est achevée, le procédé comprend une étape supplémentaire de contrôle microscopique et/ou une analyse de la matière sèche. La quantité de matière sèche et la quantité de matière lysée permettent en effet d'évaluer l'avancement de la digestion.

En pratique, on stoppe l'action des enzymes lorsque la matière sèche représente entre 2 et 15% en poids de l'extrait et lorsque la quantité de matière végétale lysée (différence entre la quantité de matière première de départ et quantité de matière végétale à l'issue de la digestion) représente moins de 20% en poids. L'arrêt de la digestion est effectué en modifiant le pH. Avantageusement, on pasteurise le produit obtenu.

Dans une dernière étape, on tamise le produit obtenu sur une maille comprise entre 150 et 500 µm. On obtient un extrait liquide qu'il convient ensuite de stabiliser pour empêcher toute action osmotique.

Le milieu stabilisant peut être à base de polyols, huiles végétales, glycol (sorbitol, propylène glycol) ou un mélange d'huile végétale ou minérale et glycérine à 50/50 par exemple.

L'extrait final se présentant sous forme liquide dans lequel sont dispersées des particules solides peut être utilisé tel quel ou sous forme sèche sur support type maltodextrine ou tout autre support adapté. L'extrait contient majoritairement des cellules lysées, de l'eau de constitution et le stabilisant.

L'invention et les avantages qui en découlent ressortiront mieux de l'exemple suivant.

### Préparation d'un extrait de bardane selon le procédé de l'invention

20 kgrs de racines de Bardane fraîches lavées sont mises en contact avec de l'azote liquide et broyées dans un cutter bilames afin d'obtenir une poudre d'une granulométrie de 500 µm. La température de la poudre est de - 60 °C.

Dans un bécher, on réalise le pied de cuve en dispersant le mélange enzymatique (CELL01) (mélange de glucanase, lignase, cellulase) mélange spécifique pour les organes sous terrains dans 2 litres d'eau distillée.

La poudre est ensuite placée dans la turbosphère 100 litres, on ajoute 20 kgrs d'un mélange d'eau /et de pied de cuve enzymatique (60/40), on mélange cette préparation pendant 10 minutes, une prise d'échantillon est réalisée pour déterminer le pH.

On rajoute 10 grs d'acide citrique pour ramener le pH à la valeur de 6.

On ramène la température du mélange vers 35 °, puis la solution enzymatique est versée dans la turbosphère. On met l'appareil en dépression à une valeur de 42 mm de Hg puis on augmente la pression à une valeur de 2 bars au moyen d'un gaz inerte. ON repère l'opération.

Après 7 heures de contact, la réaction est arrêtée en versant 150 grs d'acide citrique dans la préparation. Un échantillon est prélevé pour contrôler la lyse.

Le taux de matière sèche inférieur à 15 % et la présence de protoplastes permet de valider la fin du processus de lyse.

La préparation est ensuite tamisée sur 500 µm et mélangé avec un agent stabilisant avant d'être conditionnée.

Les avantages de l'invention ressortent bien de la description qui précède :
1- Utilisation de matériel végétal frais
2- Utilisation de gaz inerte permettant de conserver l'intégrité des composants de la matière première.
3- Broyage sous azote prévenant les risques d'oxydation
4- Processus se réalisant sous-vide partiel et sous gaz inerte sous pression, limitant les phénomènes d'oxydation.
5- Augmentation de la biodisponibilité en éliminant la paroi pectocellulosique, non digérée par l'être humain.
6- Augmentation de la concentration en éliminant toute la partie pectocellulosique de la plante par rapport à la plante sèche.
7- Conservation de l'intégrité et de l'intégralité des actifs contenus dans la matière première végétale.
8- Possibilité d'utiliser ce nouveau protocole de transformation du végétal dans les industries alimentaires, cosmétiques et pharmaceutiques.

## Revendications

1. Procédé de préparation d'un extrait végétal, comprenant les étapes suivantes :
- refroidissement de la plante ou partie de plante fraiche, à une température comprise entre -10 et -80°C,
- broyage de la plante congelée,
- mise en contact de la plante congelée broyée avec partie d'une solution enzymatique apte à digérer la paroi pectocellulosique des cellules végétales,
- ajustement du pH au pH auquel agissent les enzymes, de même que de la température à la température à laquelle agissent les enzymes,
- ajout du reste de solution enzymatique,
- application d'un cycle de dépression et de pression au moyen d'un gaz inerte,
- une fois la digestion terminée, tamisage puis stabilisation de l'extrait liquide obtenu.

2. Procédé selon la revendication 1, **caractérisé en ce que** le refroidissement consiste en une congélation ou une mise en contact de la plante fraiche avec de l'azote liquide.

3. Procédé selon la revendication 1, **caractérisé en ce que** le broyage conduit à une granulométrie de poudre comprise entre 100 et 500 µm

4. Procédé selon la revendication 1, **caractérisé en ce que** simultanément avec la mise en contact avec une partie de la solution enzymatique, la plante congelée broyée est mise en suspension dans l'eau.

5. Procédé selon la revendication 1, **caractérisé en ce que** l'eau représente 1 à 2 fois le volume de matière végétale.

6. Procédé selon la revendication 1, **caractérisé en ce que** les enzymes sont choisies dans le groupe comprenant pectinases, lignases, celluloses, β glucanases.

7. Procédé selon la revendication 1, **caractérisé en ce que** la dépression appliquée est d'une valeur minimum de 5 600 Pa.

8. Procédé selon la revendication 1, **caractérisé en ce que** la pression appliquée est d'au maximum 2 bars.

9. Procédé selon la revendication 1, **caractérisé en ce qu'**on poursuit la digestion jusqu'à ce que la matière sèche représente entre 2 et 15% en poids de l'extrait et que la quantité de matière végétale lysée représente moins de 20% en poids.

10. Procédé selon la revendication 1, **caractérisé en ce que** l'on stoppe l'action des enzymes en modifiant le pH puis on pasteurise le produit obtenu.

11. Procédé selon la revendication 1, **caractérisé en ce qu'**on tamise le produit obtenu sur une maille comprise entre 150 et 500 µm.

## Claims

1. Method for preparing a plant extract, including the following steps:
- chilling the fresh plant or plant portion to a temperature of between -10 and -80°C,
- crushing the frozen plant,
- bringing the crushed frozen plant into contact with part of an enzymatic solution capable of digesting the pectocellulosic wall of plant cells,
- adjusting the pH to the pH at which enzymes act, as well as the temperature to the temperature at which enzymes act,
- adding the remainder of the enzymatic solution,
- applying a vacuum and pressure cycle by means of an inert gas,
- once digestion is complete, filtering and then stabilising the liquid extract obtained.

2. Method as claimed in claim 1, **characterised in that** the chilling comprises freezing the fresh plant or bringing it into contact with liquid nitrogen.

3. Method as claimed in claim 1, **characterised in that** the crushing leads to a powder particle size distribution of between 100 and 500 µm.

4. Method as claimed in claim 1, **characterised in that** simultaneously with being brought into contact with a part of the enzymatic solution, the crushed frozen plant is put into suspension in water.

5. Method as claimed in claim 1, **characterised in that** water represents 1 to 2 times the volume of plant material.

6. Method as claimed in claim 1, **characterised in that** the enzymes are selected from the group that includes pectinases, lignases, cellulases, β glucanases.

7. Method as claimed in claim 1, **characterised in that** the vacuum applied has a minimum value of 5600 Pa.

8. Method as claimed in claim 1, **characterised in that** the pressure applied is a maximum of 2 bars.

9. Method as claimed in claim 1, **characterised in that** digestion continues until the dry solid matter represents between 2 and 15% by weight of the extract and the quantity of lysed plant material represents less than 20% by weight.

10. Method as claimed in claim 1, **characterised in that** the action of the enzymes is stopped by modifying the pH and the product obtained is then pasteurised.

11. Method as claimed in claim 1, **characterised in that** the product obtained is filtered through a mesh of between 150 and 500 µm.

## Patentansprüche

1. Verfahren zur Präparierung eines Pflanzenextrakts mit den nachstehenden Schritten:
- Abkühlung der Pflanze oder eines Teils der frischen Pflanze auf eine Temperatur zwischen -10 und -80°C,
- Vermahlen der tiefgefrorenen Pflanze,
- Inkontaktbringung der zermahlenen tiefgefrorenen Pflanze mit einem Teil einer Enzymlösung, die geeignet ist, die Pektozellulosewand der Pflanzenzellen zu verdauen,
- Angleichung des pH-Wertes an den pH-Wert, bei dem die Enzyme wirken, sowie der Temperatur an die Temperatur, bei der die Enzyme wirken,
- Hinzugabe der restlichen Enzymlösung,
- Anlegen eines Unterdruck- und Druckzyklus mittels eines Inertgases,
- nach beendeter Verdauung Siebung und dann Stabilisierung des gewonnenen Flüssigextrakts.

2. Verfahren nach Anspruch 1, **dadurch gekennzeichnet, dass** die Kühlung aus einem Tiefgefrieren bzw. einer Inkontaktbringung der frischen Pflanze mit flüssigem Stickstoff besteht.

3. Verfahren nach Anspruch 1, **dadurch gekennzeichnet, dass** das Zermahlen zu einer Pulvergranulometrie zwischen 100 und 500 µm führt.

4. Verfahren nach Anspruch 1, **dadurch gekennzeichnet, dass** die zermahlene tiefgefrorene Pflanze gleichzeitig mit der Inkontaktbringung mit einem Teil der Enzymlösung in Wasser suspendiert wird.

5. Verfahren nach Anspruch 1, **dadurch gekennzeichnet, dass** das Wasser 1 - 2mal dem Volumen des Pflanzenmaterials entspricht.

6. Verfahren nach Anspruch 1, **dadurch gekennzeichnet, dass** die Enzyme aus der Gruppe mit Pektinasen, Lignasen, Cellulasen, β-Glukanasen ausgewählt werden.

7. Verfahren nach Anspruch 1, **dadurch gekennzeichnet, dass** der angelegte Unterdruck mindestens einem Wert von 5600 Pa entspricht.

8. Verfahren nach Anspruch 1, **dadurch gekennzeichnet, dass** der angelegte Druck höchstens 2 bar beträgt.

9. Verfahren nach Anspruch 1, **dadurch gekennzeichnet, dass** die Verdauung fortgesetzt wird, bis die Trockensubstanz zwischen 2 und 15 Gewichtsprozente des Extrakts und das lysierte Pflanzenmaterial weniger als 20 Gewichtsprozente ausmacht.

10. Verfahren nach Anspruch 1, **dadurch gekennzeichnet, dass** die Aktion der Enzyme durch Änderung des pH-Wertes gestoppt und dann das gewonnene Produkt pasteurisiert wird.

11. Verfahren nach Anspruch 1, **dadurch gekennzeichnet, dass** das gewonnene Produkt auf einer Masche zwischen 150 und 500 µm durchgesiebt wird.
